# EUROPÄISCHE PATENTSCHRIFT

(11) **EP 0 626 151 B1**
(45) Veröffentlichungstag und Bekanntmachung des Hinweises auf die Patenterteilung: **10.09.1997**
(21) Anmeldenummer: 94107466.8
(22) Anmeldetag: 13.05.1994
(51) Int. Cl.: A61B 17/12, A61B 5/022

(54) **Kompressionsapparat zur Herstellung einer künstlichen Blutleere an Extremitäten**
Compression apparatus for creating a bloodless condition in the extremities
Appareil de compression pour provoquer un état exsangue dans les extrémités

(30) Priorität: 27.05.1993 DE 4317600
(43) Veröffentlichungstag der Anmeldung: 30.11.1994
(73) Patentinhaber: ULRICH, Heinrich, D-89077 Ulm/Donau (DE)
(72) Erfinder: ULRICH, Heinrich, D-89077 Ulm/Donau (DE)
(74) Vertreter: Fay, Hermann, Dipl.-Phys. Dr.

(56) Entgegenhaltungen:
- EP-A- 0 296 635
- US-A- 4 520 819
- UNFALLCHIRURGIE, Nr. 4, Band 12, 1986, E. EGKHER et al. "Blutdruck- abhUngige, proze gesteuerte Blutsperre zur Minimierung des Torniquet-Syndroms" Seiten 200-203

## Beschreibung

Die Erfindung betrifft einen Kompressionsapparat zur Herstellung einer künstlichen Blutleere an Extremitäten bei chirurgischen Eingriffen oder zur intravenösen Lokalanästhesie, mit einer an den Extremitäten anlegbaren, von einem Druckmedium beaufschlagbaren Kompressionsmanschette und mit einem steuerbaren Druckregelventil für das Druckmedium zur Einstellung des Kompressionsdruckes in der Kompressionsmanschette.

Kompressionsgeräte dieser Art sind aus der medizinischen Praxis bekannt und dienen zur Anlage einer Blutleere bei chirurgischen Eingriffen an den oberen oder unteren Extremitäten sowie unter Verwendung einer sog. Doppelkammer-Manschette auch zur intravenösen Lokalanästhesie. Der Kompressionsdruck sorgt distal der Kompressionsmanschette für eine auf die Operationsdauer begrenzte Blutleere. Die Größe des Kompressionsdrucks wird dabei im Rahmen eines Ermessensspielraums bestimmt, was mit dem Nachteil verbunden sein kann, daß ein zu großer Kompressionsdruck u. U. zu Läsionen, Drucklähmungen oder Spätfolgen an der betreffenden Extremität führen kann.

Der Erfindung liegt die Aufgabe zugrunde, einen Kompressionsapparat der eingangs genannten Art so auszubilden, daß ein Überschreiten zulässiger Werte für den Kompressionsdruck und damit Folgeschäden in Form von Läsionen oder Drucklähmungen vermieden werden.

Der Erfindung liegt der Gedanke bzw. die weitere Aufgabe zugrunde, den Kompressionsdruck automatisch an während der Operation wechselnde Blutdruckwerte anzupassen; steigt oder fällt während der Operation der permanent gemessene Blutdruck des Patienten, ändern die ermittelten Meßwerte entsprechend auch den jeweiligen Kompressionsdruck in der Manschette mit dem Ergebnis, daß zu hohe Kompressionsdrucke und mit ihnen verbundene Folgeschäden nicht mehr auftreten können.

Ein diesen vorgenannten Aufgaben lösender Kompressionsapparat besteht aus einem Kompressionsapparat der eingangs genannten Art in Kombination mit einem automatischen Blutdruckmeßgerät und einem Steuersignalgenerator, der ein vom laufend oder in regelmäßigen Zeitabständen ermittelten Meßwert des Blutdruckes abhängiges Steuersignal für die Steuerung des Druckregelventils erzeugt, so daß sich der Kompressionsdruck in der Kompressinsmanschette bei einer Änderung des Blutdruckmeßwertes entsprechend ändert. Ein derartiger Kompressionsapparat ist im Oberbegriff des Anspruchs 1 definiert, der aus der Zeitschrift Unfallchirurgie 12; (1986) Seiten 200-203 (Nr. 4) bekannt ist.

Die Erfindung besteht aus dem im Anspruch 1 definierten Kompressionsapparat.

In einer bevorzugten Ausführungsform der Erfindung erfolgt die Umsetzung des Blutdruckwertes in das Steuersignal für das Druckregelventil so, daß der Kompressionsdruck in der Kompressionsmanschette gleich dem aktuell gemessenen Blutdruck vergrößert um einen vorgebbaren Zusatzdruck ist. Im einfachsten Fall besitzt der Zusatzdruck zweckmäßigerweise eine vom aktuell gemessenen Blutdruck unabhängige konstante Größe; jedoch liegt es selbstverständlich auch im Rahmen der Erfindung, den Zusatzdruck in Abhängigkeit vom aktuell gemessenen Blutdruck variabel zu wählen. Im einzelnen kann die Umsetzung des Blutdruckwertes in das Steuersignal für das Druckregelventil auf verschiedene Weise erfolgen. Vorzugsweise ist der Steuersignalgenerator in ein rechnergestütztes Steuergerät integriert, das ein Rechenwerk für die Ermittlung der Steuersignalgröße aus der Größe des Blutdruckmeßwertes aufweist.

Aus Sicherheitsgründen sieht die Erfindung vor, parallel zu dem automatisch vom Steuersignalgenerator gesteuerten Druckregelventil zusätzlich ein von Hand einstellbares Druckregelventil einzusetzen und die Kompressionsmanschette durch ein Zweiwegeventil zwischen beiden Druckregelventilen umzuschalten. Fällt dann die blutdruckabhängige Steuerung des vom Steuersignalgenerator gesteuerten Druckregelventils fehlerbedingt oder aus anderen Gründen aus, schaltet sich selbsttätig das von Hand einstellbare, mechanisch arbeitende Druckregelventil ein, so daß der Kompressionsdruck in der Kompressionsmanschette keine gefährlichen Werte über- oder unterschreiten kann. Eine in diesem Zusammenhang besonders bevorzugte Ausführungsform der Erfindung ist dadurch gekennzeichnet, daß ein den Kompressionsdruck erfassender Drucksensor vorgesehen ist, der eine Umschaltung der Kompressionsmanschette auf das von Hand gesteuerte Druckregelventil veranlaßt, sobald oder solange der vom Drucksensor gemessene Kompressionsdruck (Istwert) von dem aus den Blutdruckmeßwerten ermittelten Kompressionsdruck (Sollwert) abweicht und/oder einen Alarmgeber betätigt. Zweckmäßigerweise erfolgt der Vergleich von Istwert und Sollwert im Steuergerät, das über eine Schaltleitung das Zweiwegeventil umschaltet, sobald der Istwert über eine vorgebbare, schmale Toleranzspanne hinaus vom Sollwert abweicht.

Im übrigen empfiehlt es sich, die Anordnung so zu treffen, daß das Steuergerät Anzeigeeinrichtungen für den Blutdruckmeßwert, den Zusatzdruck und/oder den Sollwert des Kompressionsdrucks und Dateneingabeeinrichtungen für die Größe des Zusatzdruckes aufweist. Außerdem empfiehlt es sich, ein den Kompressionsdruck (Istwert) an der Kompressionsmanschette anzeigendes Manometer vorzusehen.

Im folgenden wird die Erfindung an einem in der Zeichnung dargestellten Ausführungsbeispiel näher erläutert; es zeigen:
- Fig. 1: ein Prinzipschaltbild eines Kompressionsapparates nach der Erfindung,
- Fig. 2: eine Ansicht der Anzeige- und Eingabeeinrichtungen des Kompressionsapparates nach Fig. 1.

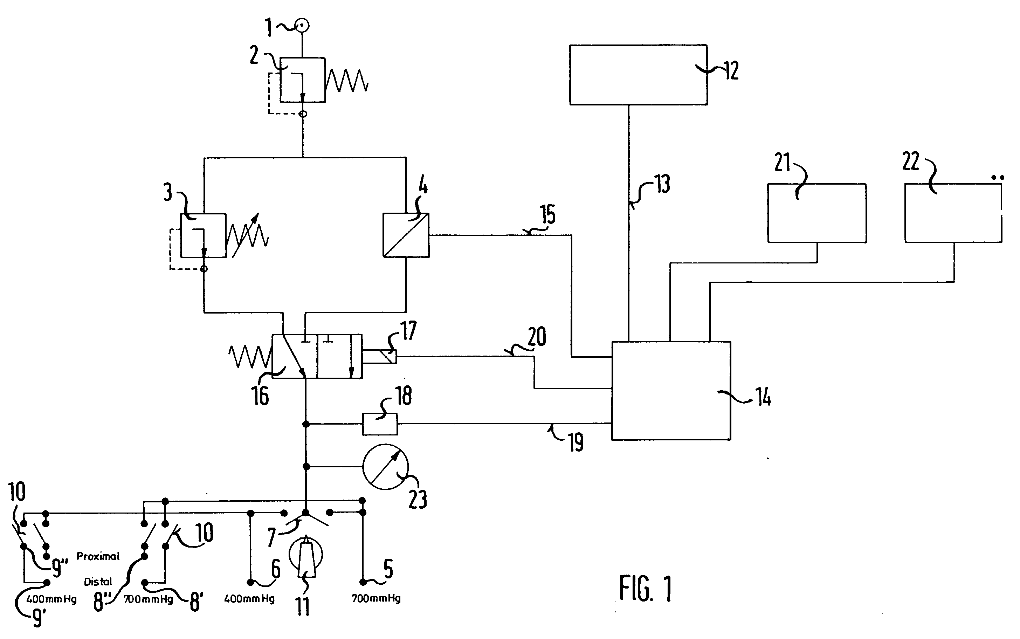

Der in Fig. 1 gezeigte Kompressionsapparat wird bei 1 an eine geeignete, selbst nicht dargestellte Druckmittelversorgung beispielsweise ein zentrales Druckluftversorgungsnetz, einen Kompressor oder eine Druckgas- insbes. CO₂-Flasche mit Druckreduzierventil angeschlossen. Ein Druckminderer 2 reduziert den Versorgungsdruck auf für den Kompressionsapparat geeignete Werte zwischen beispielsweise zwei bis fünf bar. Eine weitere Druckminderung erfolgt in stufenlos einstellbaren Druckregelventilen 3, 4, deren Ausgangsdruck, je nach Einstellung bzw. Steuerung zwischen 0 und 0,93 bar (700 mm Hg) liegen kann. An den Anschlüssen 5, 6 kann eine selbst wiederum nicht dargestellte Kompressionsmanschette angeschlossen werden, wie sie beispielsweise in DE 33 33 311 C2 beschrieben ist. Dabei ist der Anschluß 5 bis zu einem Maximaldruck von 0,93 bar (700 mm Hg) vorgesehen, so daß die Kompressionsmanschette hier anzuschließen ist, wenn sie an den unteren Extremitäten angelegt werden soll. Der andere Anschluß 6 ist im Druckwert auf 0,53 bar (400 mm Hg) beschränkt, so daß er als Anschluß für eine an den oberen Extremitäten angelegte Kompressionsmanschette dient. Mit dem mechanischen Umschalter 7 kann zwischen den Anschlüssen 5, 6 umgeschaltet werden. Bei 8', 8'', 9', 9'' kann eine sog. Doppelkammer-Manschette angeschlossen werden, die in bekannter Weise bei intravenöser Lokalanästhesie Verwendung findet. Derartige Doppelkammer-Manschetten besitzen eine proximale und eine distale Druckkammer. Für jede dieser Druckkammern ist ein eigener Anschluß 8', 9' (distal) bzw. 8'', 9'' (proximal) vorgesehen; und diese Anschlüsse sind wiederum bezüglich des maximalen Kompressionsdruckes auf Bereiche bis 0,93 bar (700 mm Hg) 8', 8'' bzw. 0,53 bar (400 mm Hg) 9', 9'' ausgelegt und durch mechanische Umschalter, die wie die Schalter 7 durch eine Handhabe 11 betätigt werden, ein-, aus- und/oder umschaltbar. Mit 12 ist ein automatisches Blutdruckmeßgerät bezeichnet, das den Blutdruck des Patienten permanent oder in regelmäßigen Zeitabständen, etwa alle 3-5 Minuten, mißt. Der der Größe des Blutdrucks entsprechende Meßwert wird über eine Leitung 13 einem Steuergerät 14 zugeführt, in dem sich ein im einzelnen nicht dargestellter Steuersignalgenerator befindet, der ein vom zugeführten Meßwert des Blutdrucks abhängiges Steuersignal für die Steuerung des Druckregelventils 4 erzeugt, wobei dieses Steuersignal über die Leitung 15 dem Steuereingang des Druckregelventils 4 zugeführt wird. Im Ausführungsbeispiel handelt es sich um ein elektronisch steuerbares Druckregelventil 4, dem entsprechend ein vom Steuersignalgenerator erzeugtes elektrisches Steuersignal zugeführt wird. Diese blutdruckabhängige Steuerung des Druckregelventils 4 hat zur Folge, daß sich der Kompressionsdruck in der Kompressionsmanschette bei einer Änderung des Blutdruckmeßwertes entsprechend ändert. Steigt der Blutdruck des Patienten, erhöht sich der Kompressionsdruck und umgekehrt. Im einfachsten Fall kann dazu die Umsetzung des Blutdruckmeßwertes 12 in das Steuersignal für das Druckregelventil 4 so erfolgen, daß der Kompressionsdruck in der Kompressionsmanschette gleich dem aktuell gemessenen Blutdruck vergrößert um einen vorgebbaren Zusatzdruck ist, so daß der Kompressionsdruck immer um den festen Betrag des Zusatzdrucks über dem aktuell gemessenen Blutdruck liegt. Im Steuergerät 14 befindet sich dazu ein im einzelnen ebenfalls nicht dargestelltes Rechenwerk, das die Steuersignalgröße aus der Größe des Blutdruckwertes ermittelt.

Parallel zu dem automatisch vom Steuersignalgenerator gesteuerten Druckregelventil 4 ist zusätzlich ein von Hand einstellbares, mechanisches Druckregelventil 3 vorgesehen.

Die Kompressionsmanschette ist durch ein Zweiwegeventil 16 zwischen beiden Druckregelventilen 3, 4 mittels eines Magnetantriebs 17 umschaltbar. Der Kompressionsdruck an der Kompressionsmanschette wird von einem Drucksensor 18 erfaßt, der die Umschaltung der Kompressionsmanschette auf das von Hand gesteuerte Druckregelventil 3 veranlaßt, wenn der vom Drucksensor 18 als Istwert gemessene Kompressionsdruck von dem aus den Blutdruckmeßwerten als Sollwert ermittelten Kompressionsdruck abweicht. Der Ausgang des Drucksensors 18 und der Steuereingang des Zweiwegeventils 16 sind über Leitungen 19, 20. mit dem Steuergerät 14 verbunden. Der Vergleich von Istwert und Sollwert des Kompressionsdrucks erfolgt im Steuergerät 14, das über die Schaltleitung 20 das Zweiwegeventil 16 umschaltet, wenn Ist- und Sollwert voneinander abweichen oder aber zumindest einen Alarmgeber betätigt.

Das Steuergerät 14 ist gemäß Fig. 1 mit Anzeigeeinrichtungen 21 für den Blutdruckmeßwert, den Zusatzdruck, den Sollwert des Kompressionsdrucks usw. ausgestattet. Außerdem sind Dateneingabeeinrichtungen 22 vorgesehen, mit denen u. a. die Größe des Zusatzdrucks eingegeben werden kann. Ein den Kompressionsdruck (Istwert) an der Kompressionsmanschette anzeigendes Manometer ist mit 23 bezeichnet.

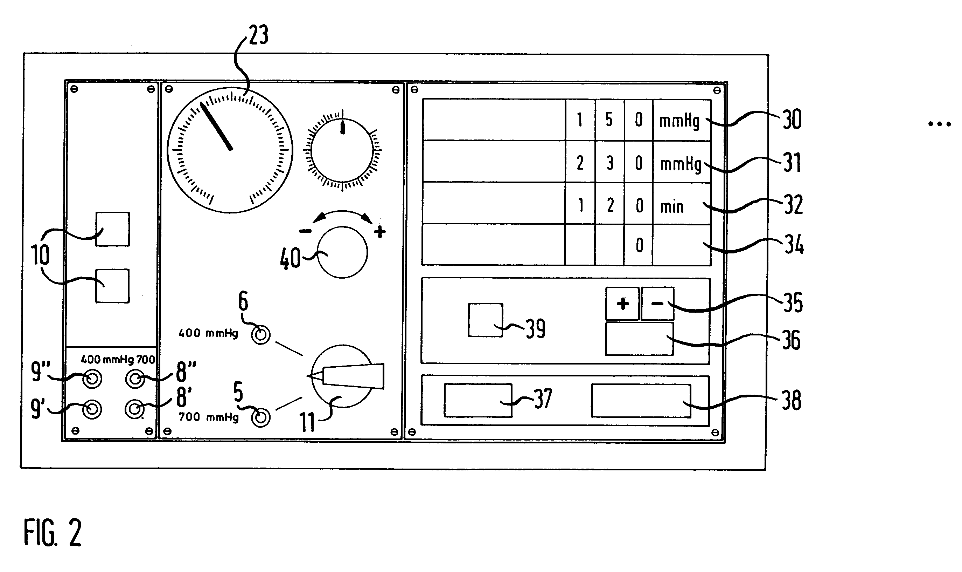

Die Drucksteuerung wird vom Steuergerät 14 in der Weise durchgeführt, daß beim Einschalten des Kompressionsapparates der Wert des vorgegebenen Zusatzdrucks zum gemessenen systolischen Blutdruckwert addiert wird und der aus dieser Summe sich ergebende Gesamtdruck als Sollwert für den Kompressionsdruck verwendet wird. Im Bedarfsfall können die Werte des Zusatzdrucks durch manuelle Eingaben nach oben oder unten variiert werden. Während der Dauer der Operation übernimmt das Steuergerät 14 die elektronische Überwachung aller relevanten Parameter; im Fehlerfall wird ein optischer und akustischer Alarm ausgelöst und in einem Fehleranzeigefeld 34 (Fig. 2) erscheint eine Code-Nummer, aus der die Art des Fehlers ersichtlich ist. Ist es nicht möglich, einen im elektronischen Bereich des Kompressionsapparates auftretenden Fehler zu beseitigen, wird die Aufrechterhaltung des Kompressionsdrucks durch das vor dem automatischen Betrieb des Kompressionsapparates zwingend eingestellte, mechanische Druckregelventil 3 gewährleistet. Dazu ist das Zweiwegeventil 16 so angeschlossen, daß bei einem Ausfall des Steuergeräts 14, insbes. auch bei einem Stromausfall, auf das von Hand einstellbare Druckregelventil 3 umgeschaltet wird.

Beim Einschalten blinken zunächst alle Anzeigen in den Feldern 30 bis 34. Dieser Anzeigentest kann durch Betätigen einer der beiden Plus- und Minustasten 35 oder der Bestätigungstaste 36 beendet werden. Daraufhin wird in dem Anzeigenfeld 30 der systolische Blutdruckwert und im Anzeigenfeld 31 der Zusatzdruckwert angezeigt, wobei diese letztere Anzeige durch zusätzliche Punkte im Anzeigenfeld 31 gekennzeichnet ist. Durch Betätigen der Tasten 35 "Plus" und "Minus" kann der Wert des Zusatzdrucks eingestellt und durch Betätigen der Taste 36 quittiert werden. Der sich nun als Summe des systolischen Blutdruckwerts und des Zusatzdruckwerts ergebende Kompressionsdruck tritt im Anzeigenfeld 31 an die Stelle des dort zuvor angezeigten Zusatzdruckwertes, jedoch nun ohne Kennzeichnung durch die Punkte im Anzeigenfeld.

Dieser Kompressionsdruck ist der Sollwert, gemäß dem das Steuergerät 14 über die Leitung 15 das Druckregelventil 4 steuert. Jedoch befindet sich vom Einschaltvorgang her noch das Zweiwegeventil 16 in der in Fig. 1 gezeigten Stellung, verbindet also die Kompressionsmanschette mit der Druckmittelversorgung über das von Hand einstellbare Druckregelventil 3. Der vom Drucksensor 18 erfaßte Istwert des Kompressionsdrucks weicht in der Regel zunächst noch von dem für die Steuerung des Druckregelventils 4 maßgebenden Sollwert ab, so daß das Zweiwegeventil 16 zunächst in der Ausgangsstellung entsprechend Fig. 1 verharrt. Diese Abweichung des Istwerts vom Sollwert wird im Anzeigenfeld 34 als Fehler angezeigt, beispielsweise mit der Ziffer "2", wenn der Druck zu niedrig, mit der Ziffer "3", wenn der Druck zu hoch ist. Nun wird der Istwert des Kompressionsdruckes durch Betätigen der Handhabe 40 des Druckregelventils 3 auf den Sollwert eingestellt, was daran erkennbar wird, daß im Anzeigenfeld 34 die Fehleranzeige erlischt. Der so eingestellte, am Manometer 23 ablesbare Druckistwert entspricht im wesentlichen dem mittleren Kompressionsdruck, um den im automatischen Betrieb des Kompressionsapparates die blutdruckabhängige Steuerung des Kompressionsdrucks erfolgt. Diese automatische Drucksteuerung wird mit der Taste 37 "Start" eingeschaltet und mit der Taste 36 quittiert. Das Zweiwegeventil 16 schaltet auf das automatisch gesteuerte Druckregelventil 4 um, so daß nun die Steuerung des Kompressionsdrucks automatisch in Abhängigkeit vom systolischen Blutdruck erfolgt. Angezeigt wird jetzt im Anzeigenfeld 30 der systolische Blutdruck, im Anzeigenfeld 31 der Sollwert des Kompressionsdrucks, im Anzeigenfeld 32 die Kompressionszeit und im Anzeigenfeld 34 ein möglicher Fehler. Dieser Betriebszustand liegt der Darstellung der Anzeigen- und
Eingabeeinrichtungen der Fig. 2 zugrunde. Zum Ausschalten der automatischen Drucksteuerung wird die Taste 38 "Stop" betätigt und mit der Taste 36 quittiert. Das Zweiwegeventil 16 schaltet daraufhin in den in Fig. 1 gezeigten Zustand zurück und der Kompressionsdruck an der Kompressionsmanschette entspricht dem am handbetätigten Druckregelventil 3 eingestellten Kompressionsdruck. Er kann nun durch Betätigen der Handhabe 40 des Druckregelventils 3 langsam auf Null zurückgestellt werden. Beim Ausfall der Elektronik kann das Gerät am Netzschalter 39 ausgeschaltet werden. Auch dann entspricht der Kompressionsdruck an der Kompressionsmanschette dem jeweils am handbetätigten Druckregelventil 3 eingestellten Wert.

## Patentansprüche

1. Kompressionsapparat zur Herstellung einer künstlichen Blutleere an Extremitäten bei chirurgischen Eingriffen oder zur intravenösen Lokalanästhesie, mit einer an den Extremitäten anlegbaren, von einem Druckmedium beaufschlagbaren Kompressionsmanschette und mit einem steuerbaren Druckregelventil (4) für das Druckmedium zur Einstellung des Kompressionsdruckes in der Kompressionsmanschette, sowie mit einem automatischen Blutdruckmeßgerät (12) und einem Steuersignalgenerator, der ein vom laufend oder in regelmäßigen Zeitabständen ermittelten Meßwert des Blutdruckes abhängiges Steuersignal für die Steuerung des Druckregelventils (4) erzeugt, so daß sich der Kompressionsdruck in der Kompressionsmanschette bei einer Änderung des Blutdruckmeßwertes entsprechend ändert, dadurch gekennzeichnet, daß parallel zu dem automatisch vom Steuersignalgenerator gesteuerten Druckregelventil (4) zusätzlich ein von Hand einstellbares Druckregelventil (3) vorgesehen und die Kompressionsmanschette durch ein Zweiwegeventil (16) zwischen beiden Druckregelventilen (3, 4) umschaltbar ist.

2. Kompressionsapparat nach Anspruch 1, dadurch gekennzeichnet, daß ein den Kompressionsdruck erfassender Drucksensor (18) vorgesehen ist, der eine Umschaltung der Kompressionsmanschette auf das von Hand gesteuerte Druckregelventil (3) veranlaßt, sobald oder solange der vom Drucksensor (18) gemessene Kompressionsdruck (Istwert) von dem aus den Blutdruckmeßwerten ermittelten Kompressionsdruck (Sollwert) abweicht, und/oder einen Alarmgeber betätigt.

3. Kompressionsapparat nach Anspruch 2, dadurch gekennzeichnet, daß der Vergleich von Istwert und Sollwert in einem rechnergestützten Steuergerät (14) erfolgt, das ein Rechenwerk für die Ermittlung der Steuersignalgröße aus der Größe des Blutdruckmeßwertes, vergrößert um einen vorgebbaren Zusatzdruck, aufweist, und daß das Steuergerät (14) über eine Schaltleitung (20) das Zweiwegeventil (16) umschaltet.

4. Kompressionsapparat nach Anspruch 3, dadurch gekennzeichnet, daß das Steuergerät (14) Anzeigeeinrichtungen (21, 30 bis 34) für den Blutdruckmeßwert, den Zusatzdruck und/oder den Sollwert des Kompressionsdrucks und Dateneingabeeinrichtungen (22, 35) für die Größe des Zusatzdruckes aufweist.

5. Kompressionsapparat nach einem der Ansprüche 1 bis 4, dadurch gekennzeichnet, daß ein den Kompressionsdruck (Istwert) an der Kompressionsmanschette anzeigendes Manometer (23) vorgesehen ist.

## Claims

1. A compression apparatus for producing artificial bloodlessness at extremities in surgical operations or for intravenous local anaesthesia, comprising a compression cuff which can be applied to the extremities and which can be acted upon by a pressure medium, a controllable pressure regulating valve (4) for the pressure medium for setting the compression pressure in the compression cuff, and an automatic blood pressure measuring device (12) and a control signal generator which produces a control signal for control of the pressure regulating valve (4), which control signal is dependent on the blood pressure measurement value which is ascertained continuously or at regular time intervals, so that the compression pressure in the compression cuff is correspondingly altered upon an alteration in the blood pressure measurement value, characterised in that in parallel with the pressure regulating valve (4) which is automatically controlled by the control signal generator, there is additionally a manually settable pressure regulating valve (3) and the compression cuff can be switched over between the two pressure regulating valves (3, 4) by a two-way valve (16).

2. A compression apparatus according to claim 1 characterised in that there is provided a pressure sensor (18) for detecting the compression pressure, which causes the compression cuff to be switched over to the manually controlled pressure regulating valve (3) as soon as or as long as the compression pressure measured by the pressure sensor (18) (the actual value) differs from the compression pressure ascertained from the blood pressure measurement values (the reference value), and/or actuates an alarm device.

3. A compression apparatus according to claim 2 characterised in that the comparison of actual value and reference value is effected in a computer-aided control device (14) which has a computing means for determining the control signal magnitude from the magnitude of the blood pressure measurement value, increased by a predeterminable additional pressure, and that the control device (14) switches over the two-way valve (14) by way of a switching line (20).

4. A compression apparatus according to claim 3 characterised in that the control device (14) has display devices (21, 30 to 34) for the blood pressure measurement value, additional pressure and/or the reference value in respect of the compression pressure and data input devices (22, 35) for the magnitude of the additional pressure.

5. A compression apparatus according to one of claims 1 to 4 characterised in that there is provided a manometer (23) for displaying the compression pressure (actual value) at the compression cuff.

## Revendications

1. Appareil de compression pour créer artificiellement un état de circulation sanguine réduite au niveau d'extrémités lors d'interventions chirurgicales ou pour l'anesthésie locale intraveineuse, comportant un brassard de compression qui est posé au niveau des extrémités et est alimenté avec un fluide de pression, une valve (4) commandée de réglage de la pression du fluide de pression pour le réglage de la pression de compression dans le brassard ainsi qu'un appareil (12) automatique de mesure de la tension artérielle et un générateur de signal de commande qui, pour la commande de la valve (4) de réglage de pression, produit un signal de commande dépendant de la tension artérielle mesurée en continu ou à intervalles réguliers, de telle sorte qu'en cas de variation de la tension artérielle, la pression de compression dans le brassard de compression soit modifiée en conséquence, caractérisé par le fait qu'une valve (3) de réglage de pression, réglable manuellement, est prévue en supplément en parallèle avec la valve (4) de réglage de pression commandée de manière automatique par le générateur de signaux de commande et que le brassard de compression peut être connecté à l'une ou à l'autre des deux valves de réglage de pression (3, 4) à l'aide d'un distributeur (16) à deux voies.

2. Appareil de compression selon la revendication 1, caractérisé par le lait qu'il est prévu un capteur de pression (18) qui mesure la pression de compression et qui déclenche une commutation du brassard de compression vers la valve (3) manuelle de réglage de pression dès que ou tant que la pression de compression (valeur effective) mesurée par le capteur de pression (18) s'écarte de la pression de compression (pression de consigne) déterminée à partir des valeurs de tension artérielle et/ou qui active un générateur d'alarme.

3. Appareil de compression selon la revendication 2, caractérisé par le fait que la comparaison valeur effective - valeur de consigne a lieu dans un appareil de commande (14) assisté par ordinateur qui comporte un calculateur pour la détermination de la valeur du signal de commande à partir de la valeur mesurée de la tension artérielle augmentée d'une pression additionnelle prédéterminée et par le fait l'appareil de commande (14) commute le distributeur (16) deux voies par le biais d'une ligne de commutation (20).

4. Appareil de compression selon la revendication 3, caractérisé par le fait que l'appareil de commande (14) comporte des dispositifs (21, 30 à 34) d'affichage pour la tension artérielle mesurée, pour la pression additionnelle et/ou pour la valeur de consigne de la pression de compression et des dispositifs (22, 35) d'entrée de données pour la valeur de la pression additionnelle.

5. Appareil de compression selon l'une des revendications 1 à 4, caractérisé par le fait qu'un manomètre (23) indiquant la pression de compression (valeur effective) est prévu sur le brassard de compression.
